(19) Europäisches Patentamt

European Patent Office

Office européen des brevets

(11) EP 1 586 268 A2

(12) EUROPEAN PATENT APPLICATION

(43) Date of publication:
19.10.2005 Bulletin 2005/42

(51) Int Cl.⁷: A61B 5/15

(21) Application number: 05252403.0

(22) Date of filing: 18.04.2005

(84) Designated Contracting States:
AT BE BG CH CY CZ DE DK EE ES FI FR GB GR
HU IE IS IT LI LT LU MC NL PL PT RO SE SI SK TR
Designated Extension States:
AL BA HR LV MK YU

(30) Priority: 16.04.2004 US 825899

(71) Applicant: LIFESCAN, INC.
Milpitas, California 95035 (US)

(72) Inventors:
• Allen, John J.
Mendota Heights Minnesota 55118 (US)
• Menendez, Adolfo
Cottage Grove Minnesota 55016 (US)

(74) Representative: Mercer, Christopher Paul et al
Carpmaels & Ransford,
43-45 Bloomsbury Square
London WC1A 2RA (GB)

(54) **Lancet**

(57)    An apparatus (100) for extracting bodily fluid (e.g., whole blood) from a user's finger includes a housing (102) with a lancing mechanism (104) and a clamping mechanism (106) attached to thereto. The clamping mechanism includes a lower arm assembly (108) and an upper arm assembly (110). The upper and lower arm assemblies are operatively connected such that when a user's finger applies a user force to the lower arm assembly and displaces the lower arm assembly from a first to a second position, the upper and lower arm assemblies cooperate to engage the user's finger with a compressive force that is greater than the user force. In addition, the lancing mechanism is configured to lance a target site on the user's finger while the upper and lower arm assemblies are cooperating to engage the user's finger. Thereafter, the compressive force serves to extract a bodily fluid sample from the lanced target site.

FIG. 1

EP 1 586 268 A2

## Description

### BACKGROUND OF THE INVENTION

#### 1. Field of the Invention

The present invention relates, in general, to fluid extraction apparatus and, in particular, to apparatus for extracting bodily fluid and associated methods.

#### 2. Description of the Related Art

A variety of medical conditions, such as diabetes, call for the monitoring of an analyte concentration (e.g., glucose concentration) in a blood, interstitial fluid or other bodily fluid sample. Typically, such monitoring requires the extraction of a bodily fluid sample from a target site (e.g., a dermal tissue target site) on a user's finger.

The extraction (also referred to as "expression") of a blood sample from a user's finger generally involves lancing the dermal tissue target site and applying pressure in the vicinity of the lanced site to express the blood sample. In the circumstance that the dermal tissue target site is on a user's finger, it is known to extract the blood sample using a fluid extraction device with a pressure ring. The pressure ring is employed to apply pressure against the dermal tissue target site either prior to, and/or after, lancing.

To reduce user pain or discomfort during lancing, it can be desirable to lance the target site to a relatively shallow penetration depth (such as a penetration depth in the range of 0.5 mm to 1.0 mm). However, expressing a bodily fluid sample from a target site that has been lanced to a relatively shallow penetration depth requires a greater amount of applied pressure than expressing from a target site that has been lanced to a relatively deep penetration depth. The strength and dexterity necessary to apply the required pressure (e.g., an applied pressure of 15N or more around a dermal tissue target site at the end of a finger) can be lacking in some users.

In it known to employ various extraction apparatus during lancing that aid in the application of pressure. However, these apparatuses are typically cumbersome and complicated to operate (e.g., requiring a two-handed operation), function in a non-intuitive manner, and/or utilize expensive and bulky motorized components.

Still needed in the field, therefore, is an apparatus for extracting bodily fluid from a target site that facilitates the application of pressure to the target site, yet is simple and intuitive to operate. Furthermore, the apparatus should be compact and not require the use of expensive and/or bulky motorized components. Also needed is a process for extracting a bodily fluid sample that is simple and intuitive.

### SUMMARY OF THE INVENTION

Apparatus for extracting bodily fluid according to exemplary embodiments of the present invention facilitate the application of pressure to a target site, yet are simple and intuitive to operate. Furthermore, the apparatus are compact and do not require the use of expensive and/or bulky motorized components. Certain embodiments can be operated with one hand and without the separate actuation of motors or other bulky components.

An apparatus for extracting bodily fluid according to an exemplary embodiment of the present invention includes a housing, a lancing mechanism attached to the housing and a clamping mechanism attached to the housing. The clamping mechanism includes an upper arm assembly and a lower arm assembly.

The upper and lower arm assemblies are operatively connected such that when a user's finger applies a predetermined user force to the lower arm assembly and displaces the lower arm assembly from a first position to a second position, the upper arm assembly and lower arm assembly cooperate to engage the user's finger with a compressive force that is greater than the predetermined user force. In addition, the lancing mechanism is configured to lance a target site on the user's finger while the upper arm assembly and lower arm assembly are cooperating to engage the user's finger. Thereafter, the compressive force serves to extract a bodily fluid sample from the lanced target site.

The upper and lower arm assemblies can be operatively connected by, for example, a mechanical linkage(s) that employ mechanical advantage to couple the predetermined user force with the compressive force. Because of the mechanical advantage of the mechanical linkage, the compressive force is greater than the predetermined user force. Exemplary embodiments of apparatus for extracting bodily fluid according to the present invention can optionally include a force limiting means (such as a force limiting spring) that prevents the compressive force from exceeding a predetermined level.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings (wherein like numerals represent like elements), of which:

FIG. 1 is a simplified perspective view of an apparatus for extracting bodily fluid according to an exemplary embodiment of the present invention;
FIG. 2A is a simplified perspective view of a portion of the apparatus illustrated in FIG. 1;

FIG. 2B is a simplified cross-sectional view of the portion illustrated in FIG. 2A;

FIG. 2C is a simplified side-view of the fluid portion shown in FIGs. 2A and 2B with a user's finger engaged therein;

FIGs. 3A and 3B are simplified cross-sectional depictions of a user's finger engaged with only a lower arm assembly and with both an upper compression surface and a lower arm assembly, respectively;

FIG. 4 is a simplified side-view of the apparatus of FIG. 1 with a user's finger engaged therein and the upper and lower arm assemblies in a second position;

FIGS. 5A and 5B are simplified side views of the apparatus shown in FIG. 1 with the upper arm assembly fully rotated counter clockwise with a user's finger engaged with the lower arm assembly and with a user's finger depressing the lower arm assembly, respectively;

FIGS. 6A and 6B are a side view and an end view, respectively, of the embodiment shown in FIG. 1 in a storage configuration;

FIGs. 7A through 7C are schematic side views depicting a sequence of steps in the operation of the apparatus of FIG. 1;

FIGs. 8A and 8B are simplified perspective views of an apparatus for extracting bodily fluid according to another exemplary embodiment of the present invention;

FIG. 8C is a simplified cross-sectional view of the apparatus of FIGs. 9A and 9B;

FIG. 9 is a simplified schematic cross-sectional view of an apparatus for extracting bodily fluid according to yet another exemplary embodiment of the present invention;

FIG. 10 is a simplified schematic cross-sectional view of an apparatus for extracting bodily fluid according to still another exemplary embodiment of the present invention; and

FIG. 11 is a flow chart depicting a sequence of steps in a process according to the present invention.

**DETAILED DESCRIPTION OF THE INVENTION**

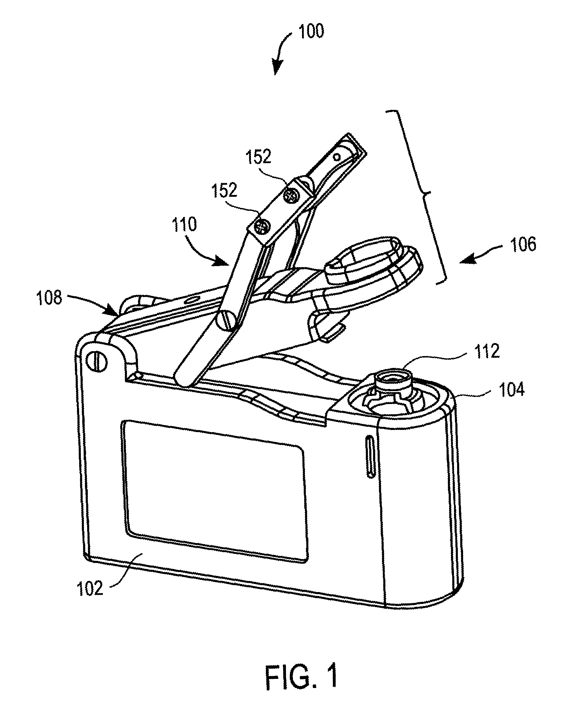

FIGs. 1, 2A, 2B, 2C and 4 are various depictions of an apparatus 100 for extracting bodily fluid (e. g., whole blood) from a target site (such as a dermal tissue target site on a user's finger) according to the present invention. Apparatus 100 includes a housing 102, a lancing mechanism 104 attached to housing 102 and a clamping mechanism 106 also attached to housing 102. Clamping mechanism 106 includes a lower arm assembly 108 and an upper arm assembly 110.

Lancing mechanism 104 can include means for measuring, analyzing and displaying an analyte concentration of a bodily fluid sample extracted by apparatus 100. However, once apprised of the present invention, those skilled in the art will recognize that embodiments of the present invention can also be used for extracting a bodily fluid sample for subsequent testing by a separate analysis system. Any suitable lancing mechanism can be employed in apparatus according to the present invention. An example of a suitable lancing mechanism is described in US. Patent No. 6,197,040, which is hereby fully incorporated herein by reference.

In the embodiment of FIGs. 1, 2A-2C and 4, lancing mechanism 104 includes a skin probe 112 and a dermal tissue penetration member (not shown). Skin probe 112 is configured to limit the depth to which the dermal tissue penetration member can penetrate a target site (e.g., a dermal tissue target site) when apparatus 100 is employed to extract a bodily fluid sample. Any suitable skin probe known to one skilled in the art can be employed in embodiments of the present invention. A non-limiting example of a suitable skin probe is described in co-pending U.S. Patent Application No. 10/690,083. In addition, skin probe 112 can be either moveable or fixed relative to housing 102.

Dermal tissue penetration members employed in embodiments of the present invention can be a conventional lancet, as is known to those skilled in the art, or can be part of an integrated medical device that includes a dermal tissue penetration member and a test strip, examples of which are described in International Application No. PCT/GB01/05634 (published as WO 02/49507 on June 27, 2002) and U.S. Patent Application No. 10/143,399, both of which are fully incorporated herein by reference.

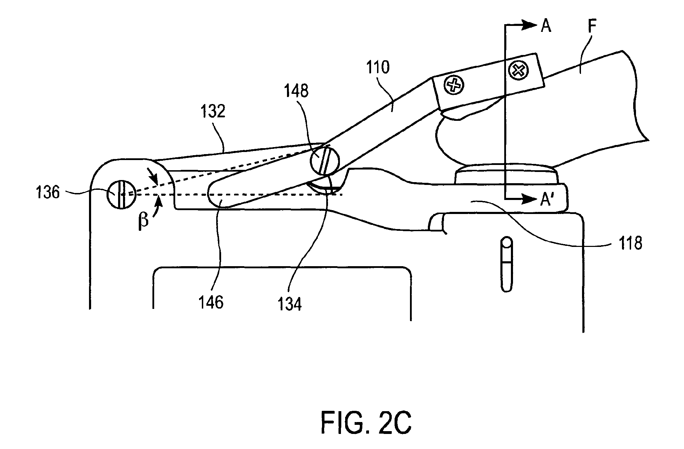

As explained in detail below, upper and lower arm assemblies 110 and 108 are operatively connected such that when a user's finger applies a predetermined user force to lower arm assembly 108 and displaces lower arm assembly 108 from a first position to a second position (depicted in FIG. 2C), upper arm assembly 110 and lower arm assembly 108 cooperate to engage the user's finger with a compressive force that is greater than the predetermined user force. In addition, lancing mechanism 104 is configured to lance a target site on the user's finger while upper arm assembly 110 and lower arm assembly 108 are cooperating to engage the user's finger. Thereafter, the compressive force serves to extract a bodily fluid sample from the lanced target site.

In the embodiment of FIGs. 1, 2A, 2B, 2C and 4, clamping mechanism 106 is pivotally connected to housing 102 and configured to allow one-handed operation of apparatus 100 with relatively effortless user force while facilitating the extraction of a bodily fluid sample (e.g., a blood sample) out of a lanced target site (such as a lanced dermal tissue target site) without manipulation (e.g., squeezing and/or milking) of the target site subsequent to lancing. Indeed, as described hereinafter, clamping mechanism 106 is configured such that a predetermined force applied by a user's finger is sufficient to operate apparatus 100.

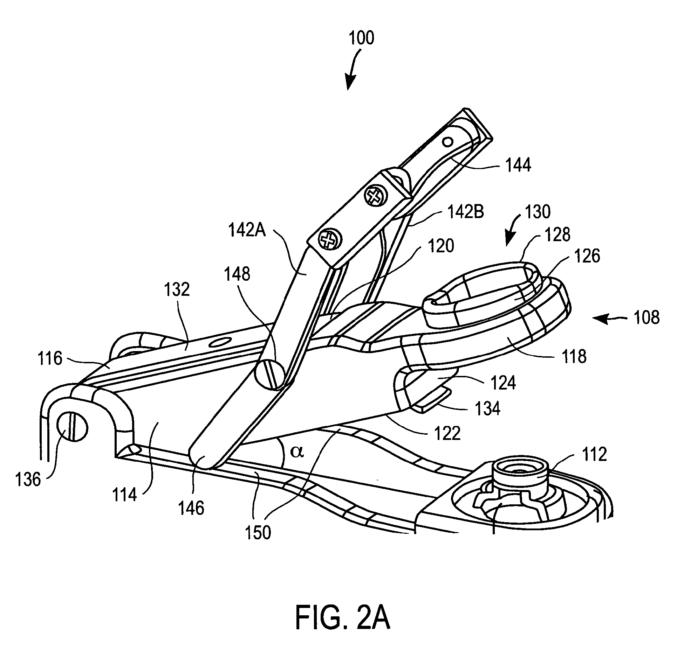

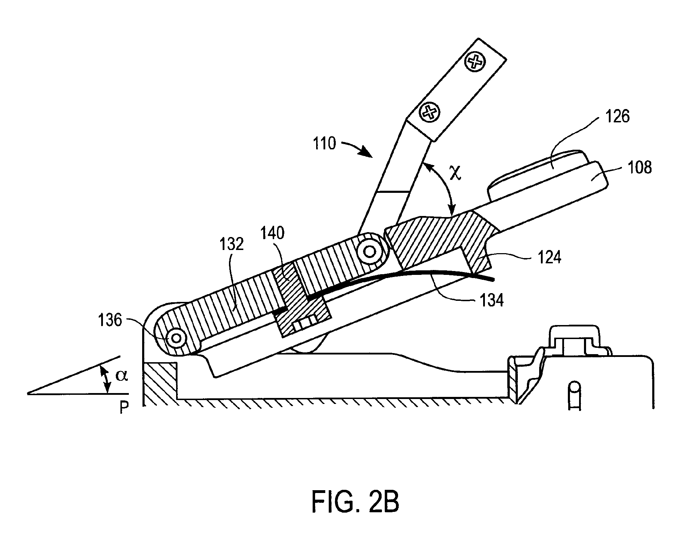

Referring in particular to FIGs. 2A, 2B and 2C, lower arm assembly 108 includes a body 114 with a proximal end 116, a distal end 118, a body top surface 120 and a body bottom surface 122. Distal end 118 is configured to accommodate the shape of lancing mechanism 104 and can be, for example, step-shaped in cross-section. Distal end 118 includes a lower lip 124 and a pressure ring 126 for engaging a target site (e.g., a dermal tissue target site of a user's finger F as depicted in FIG. 2C). Pressure ring 126 includes a rim 128 surrounding an opening 130 for skin probe 112 the to extend through. Rim 128 can be, for example, flat, raised and/or contoured to accommodate different target sites. Opening 130 can be any suitable shape including, but not limited to, circular, oval, square, triangular, hexagonal and octagonal shapes.

Pressure ring 126 can be removable or permanently attached to body 114. Non-limiting examples of pressure rings that can be employed in embodiments of the present invention are described in U.S. Patent Application Nos. 09/877514 (published as US 2002/0016606 on February 7, 2002) and 10/653,023, both of which are hereby fully incorporated by reference. Pressure ring 126 can be formed of relatively rigid plastic material including, but not limited to, polystyrene, polycarbonate and polyester, or of relatively resiliently deformable material including, but not limited to, elastomeric materials, polymeric materials, polyurethane materials, latex materials, silicone materials and any combinations thereof.

Lower arm assembly's proximal end 116 includes a means for limiting the compressive force applied to user's finger F, namely a force limiting arm 132 and a force limiting spring 134 that are operatively connected to limit the compressive force. Force limiting arm 132 is nested within body 114 and extends from lower arm assembly's proximal end 116 to approximately the center of body 114.

Force limiting arm 132 and lower arm assembly 108 are pivotally attached to housing 102 at lower pivot axis 136. Force limiting arm 132 is also pivotally attached to approximately the center of upper arm assembly 110. Force limiting spring 134 is attached to force limiting arm 132 by a screw 140 or other suitable means such as a weld or adhesive. Body 114 and force limiting arm 132 can be formed, for example, of rigid materials including polycarbonate, polystyrene or metal.

The angle α formed between lower arm assembly 108 and plane P (see, for example, FIG. 2B) can range from about 0 to about 45 degrees during operation of apparatus 100. Lower arm assembly's distal end 118 contacts housing 102 when α is approximately 0 degrees.

Referring to FIG. 2B, force limiting spring 134 extends from about the center of force limiting arm 132 internally through body 114 of lower arm assembly 108 and exits body 114 at lower lip 124 such that when α is about 0 degrees and a user's finger F is engaged (see FIG. 2C), force limiting arm 132 rotates counterclockwise about lower pivot axis 136, causing force limiting spring 134 to deflect against lower lip 124. Force limiting spring 134, therefore, beneficially limits the amount of compressive force applied to a user's finger F engaged in clamping mechanism 106. In such a circumstance, angle β is formed between force limiting arm 132 and lower arm assembly's body 114 (see FIG. 2C). Angle β can range from, for example, approximately 0 degrees to 20 degrees. In general terms, the purpose of force limiting spring 134 (or other suitable means for limiting the compressive force as would be known to one skilled in the art once apprised of the present disclosure) is to limit the constrictive force applied by the upper and lower arm assemblies to a user's finger. This can be accomplished, for example, by providing for the upper and lower arm assemblies to deform and/or deflect in a manner that mitigates the compressive force that would otherwise occur in a completely rigid clamping mechanism.

Upper arm assembly 110 includes substantially parallel upper arms 142A and 142B and an upper compression surface 144 (see, for example, FIG. 2A). Upper arms 142A and 142B each include a cam portion 146 and are each pivotally attached to force limiting arm 132 at upper pivot axis 148. An angle χ formed between lower arm assembly 108 and upper arms 142A, 142B (shown in FIG. 2B) can range from 0 degrees to 180 degrees during operation of apparatus 100. Cam portions 146 are in contact with sliding surfaces 150 of housing 102. When upper arms 142A, 142B rotate about upper pivot axes 148, cam portions 146 slide along sliding surfaces 150.

Upper compression surface 144 applies pressure against the top of user's finger F when user's finger F is engaged with clamping mechanism 106 and thereby aids in the extraction of bodily fluid from user's finger F. Upper compression surface 144 can be any suitable upper compression surface including, but not limited to, a curved upper compression surface, an angled upper compression surface, a multi-sided upper compression surface or the surface of two cylindrical bushings. Furthermore, upper compression surface 144 can be formed of flexible material including, but not limited to, leather, artificial leather, nylon strapping, rubber, or a semi-rigid plastic such as vinyl or polypropylene.

In the embodiment illustrated in FIGs. 1, 2A, 2B and 2C, upper compression surface 144 is removably attached to upper arms 142A, 142B by screws 152. Upper compression surface 144 can also be adhered to upper arms 142A, 142B by techniques known to those skilled in the art, including double-sided heat-sealed gluing or double-sided pressure sensitive adhesion. Upper compression surface 144 can also be sewn or riveted onto upper arms 142A, 142B.

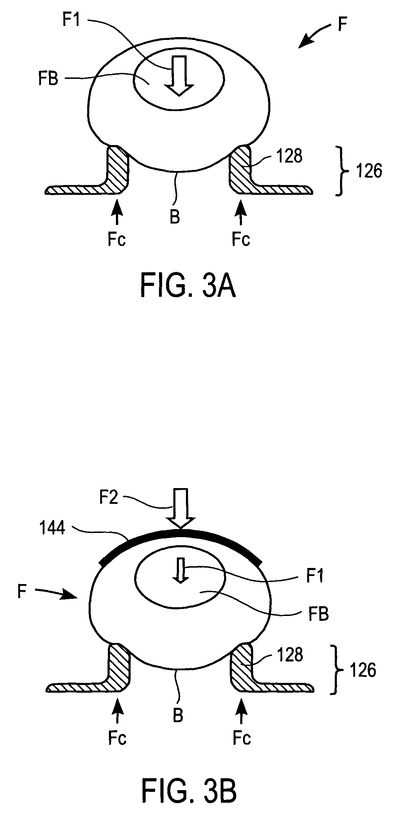

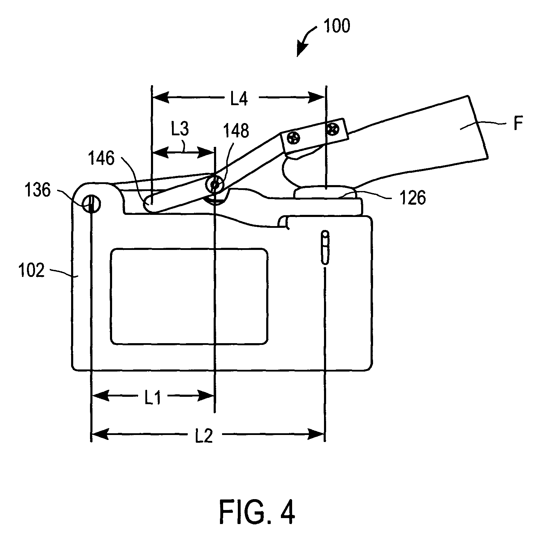

It is postulated, without being bound, that the manner in which the compressive force is applied by apparatus according to exemplary embodiments of the present invention can be explained by the following description, which references FIGs. 3A and 3B. Referring to FIG. 3A, as user's finger F is urged toward pressure ring 126, a downward force F1 is created by finger bone FB of user's finger F. Pressure ring's rim 128 reacts with an equal and opposite force Fc against the bottom side of user's finger F. As F1 increases, a pressurized skin bulge B can be created. Application of a compressive force F2 by upper compression surface 144 to the top of user's finger F enables force F1 to be reduced while maintaining compressive force Fc and skin bulge B, as shown in FIG. 4B. Thus, the amount of user force that must be applied by user's finger F to maintain skin bulge B is beneficially reduced by the application of compressive force F2 by upper compression surface 144, thereby reducing discomfort to, and effort required by, a user.

Referring now to FIG. 4, compressive force F2 generated by the upper arm assembly's upper compression surface can be defined by the following equations:

$$Fc = F1 + F2$$

and

$$F2 = Fc * (L2/L1)*(L3/L4)$$

where:

Fc is the compressive force on a user's finger resulting from cooperation of the upper arm assembly and the lower arm assembly;

L1 is the distance from lower pivot axis 136 to upper pivot axis 148;

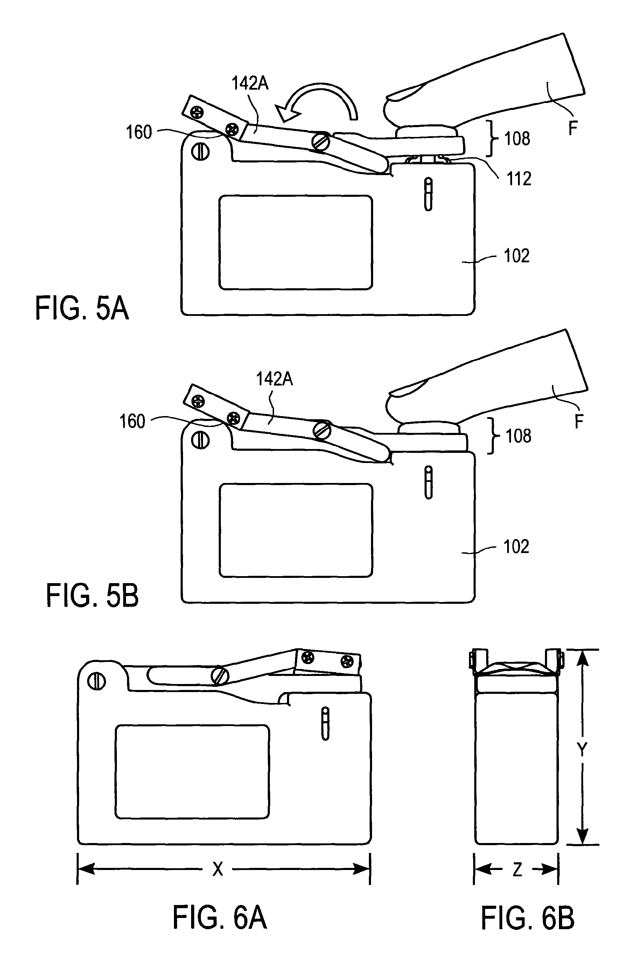

L2 is the distance from lower pivot axis 136 to the centerline of pressure ring 126 (which in FIG. 5 is operatively aligned with upper compression surface 144);

L3 is the distance from either cam portion 146 to upper pivot axis 148; and

L4 is the distance from either cam potions 146 to the centerline of pressure ring 126.

Distances L1, L2, L3 and L4 can be, for example, in the ranges of about 15 mm to 32 mm, 30 mm to 60 mm, 8 mm to 16 mm and 22 mm to 44 mm, respectively. In an exemplary embodiment in which L1 = 32 mm, L2 = 60 mm, L3 = 16 mm and L4 = 44 mm, 68% of the compressive force Fc is generated by upper compression surface 144 of clamping mechanism 106 (i.e., F2) and 32% of the compressive force Fc is generated by a user's finger F (i.e., F1).

In general terms, apparatus for extracting bodily fluid according to embodiments of the present invention are configured such that movement of the lower arm assembly from a first position to a second position is translated into movement of the upper arm assembly in the same direction as the lower arm assembly such that the distance between the upper and lower arm assemblies is decreased. Moreover, a mechanical advantage is provided when this configuration provides for a portion of Fc to be provided by F2.

The compressive force Fc required to successfully extract a bodily fluid sample from a dermal tissue target site of a user's finger can be as high as approximately 18N. A user can experience discomfort when applying a force F1 that is greater than 10N. From the above example, the force required by user's finger for the exemplary embodiment can be up to about 6N, which is less than the force at which a user typically experiences discomfort. Thus, apparatus 100 beneficially decreases the amount of force required by a user to successfully extract bodily fluid.

FIGs. 5A and 5B depict side views of apparatus 100 with upper arms 142A and 142B rotated counterclockwise (in the direction of the open arrow of FIG. 5A) about upper pivot axis 148. In the configuration of FIGs. 5A and 5B, upper arms 142A and 142B contact stop surface 160 on housing 102. In FIG. 5A, lower arm assembly 108 is elevated slightly above skin probe 112. Additional force from user's finger F is required to rotate lower arm assembly 108 clockwise to fully engage skin probe 112 with dermal tissue of user's finger F, as shown in FIG. 5B. In the configuration of FIGs. 5A and 5B, force limiting arm 132 remains stationary and the force required to urge lower arm assembly 108 onto skin probe 112 is equal to a biasing force created by force limiting spring 134. Operation of apparatus 100 in the manner depicted in FIGs. 5A and 5B does not involve the application of force to a user's finger by upper compression surface 144. However, with upper arms 142A and 142B in the position of FIGs. 5A and 5B, apparatus 100 can be employed to extract bodily fluid from target sites other than a target site of a user's finger.

FIGs. 6A and 6B show side and end views, respectively, of apparatus 100 in a storage configuration. In FIGs. 6A and 6B, upper arm assembly 110 is fully rotated clockwise about upper pivot axis 148 and lower arm assembly 108 is fully rotated clockwise about lower pivot axis 136. In such a configuration, apparatus 100 is compact and can fit, for example, in the palm of a user's hand. Typical non-limiting dimensions depicted in FIGs. 6A and 6B are an X dimension of 77mm, a Y dimension of 53 mm and a Z dimension of 22 mm.

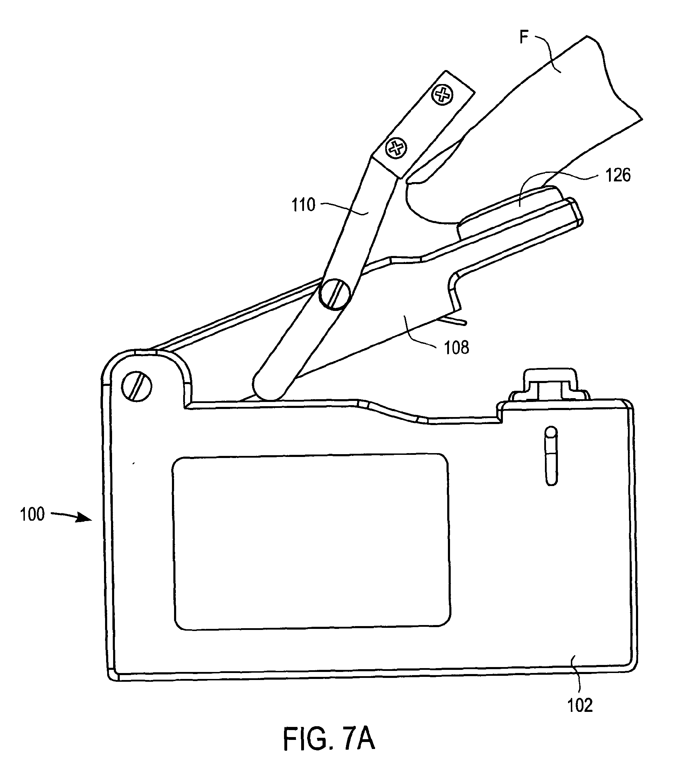

FIGs. 7A through 7C are schematic side views depicting a sequence of steps in the operation of the apparatus 100 of FIG. 1. FIG. 7A depicts upper and lower arm assemblies 110 and 108 in a first position with a user's finger F contacting pressure ring 126 of lower arm assembly 108 but not applying any significant force. The upper and lower arm assemblies can be held in the first position by, for example, the user's finger, nominal (in comparison to F1) friction around lower pivot axis 136, or nominal (in comparison to F1) spring bias against lower arm assembly 108.

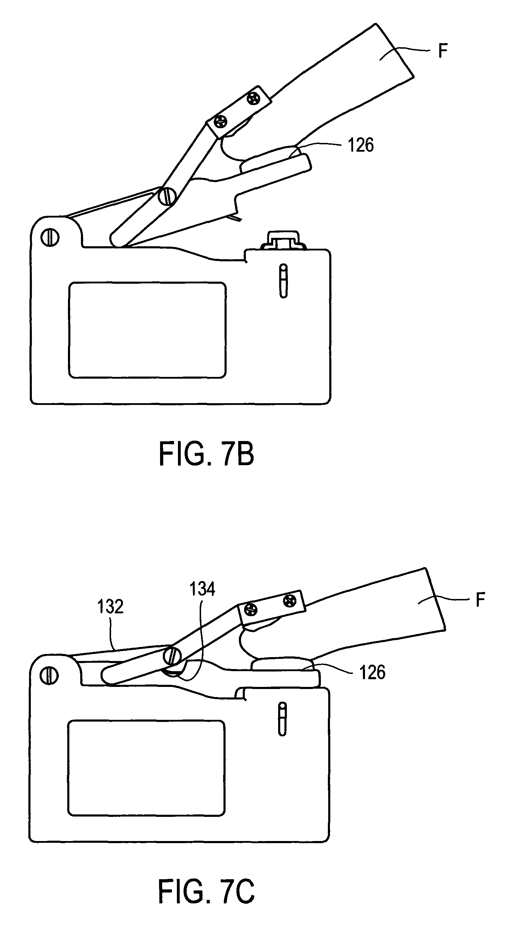

FIG. 7B depicts lower arm assembly 108 rotating clockwise under an applied force by user's finger F. As lower arm assembly 108 rotates, upper arm assembly 110 engages user's finger F (see FIG. 7B). Once user's finger F has applied a predetermined force, upper and lower arm assemblies 108 and 110 reach a second position depicted in FIG. 7C. At this second position, upper compression surface 144 and pressure ring 126 cooperate to exert a compressive force on user's finger F. The compressive force applied to is the sum of the forces applied by upper compression surface 144 and the predetermined user's force and is typically in the range of, for example, about 9N to 18N.

Typically, the compressive force applied by compression surface 144 (i.e., F2) is greater than or equal to the predetermined force applied by user's finger F (i.e., F1). F2, however, need not be greater than F 1 in order to provide benefits as described herein. For the example described above, about 68% of the total compressive force (Fc) is contributed by upper compression surface 144 (i.e., F2), whereas about 32% of the total force is contributed by the predetermined user's force (i.e., F1).

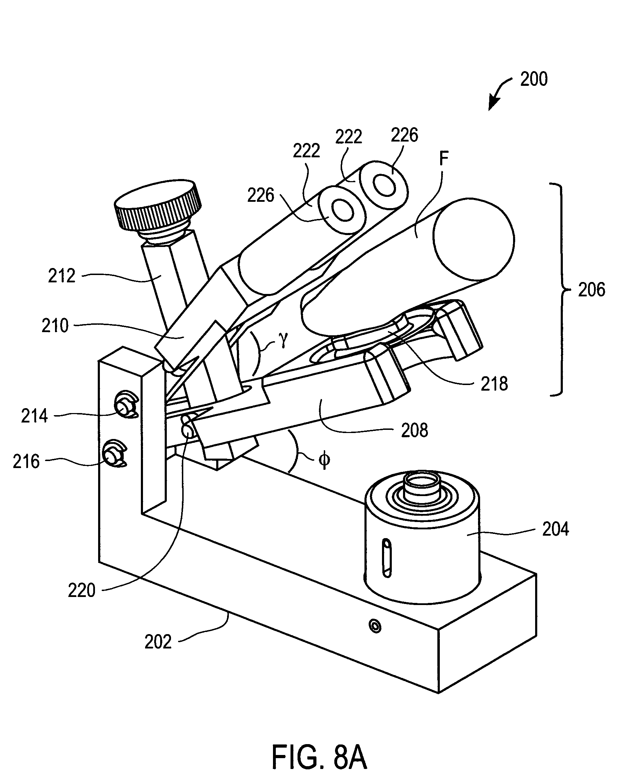

FIGs. 8A through 8C depict various views of apparatus 200 for extracting bodily fluid according to another exemplary embodiment of the present invention. Apparatus 200 includes a housing 202, a lancing mechanism 204 and a clamping mechanism 206. Clamping mechanism 206 includes a lower arm assembly 208, an upper arm assembly 210 and a linking bar 212.

Upper arm assembly 210 and lower arm assembly 208 are pivotally attached to housing 202 by upper pivot axis 214 and a lower pivot axis 216, respectively. Angle φ formed between lower arm assembly 208 and housing 202 can vary from about 0 degrees to about 45 degrees during operation of apparatus 200. Angle γ formed between upper arm assembly 210 and lower arm assembly 208 can vary from about 0 degrees to about 30 degrees during operation of apparatus 200.

Lower arm assembly 208 includes a pressure ring 218 and a linking bar lower pivot axis 220. Lower arm assembly 208 is pivotally attached to linking bar 212 at linking bar lower pivot axis 220.

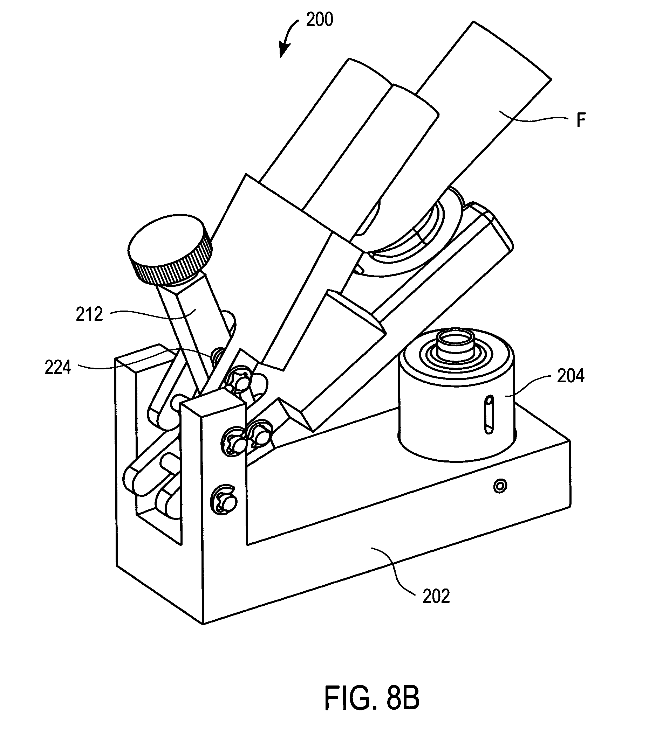

Upper arm assembly 210 includes two cylindrically-shaped upper compression surfaces 222 and a linking bar upper pivot axis 224, as illustrated in FIG. 8B. Upper arm assembly 210 is pivotally attached to linking bar 212 at linking bar upper pivot axis 224.

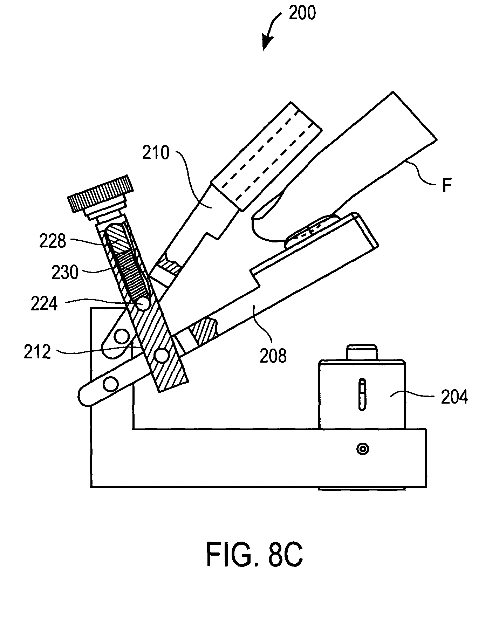

In the embodiment of FIGs. 8A, 8B and 8C, upper compression surfaces 222 are surfaces of removable cylindrical bushings 226. Furthermore compression surfaces 222 are configured to engage the top of user's finger F.

Referring to FIG. 8C, linking bar 212 includes an adjustment screw 228 in contact with a force limiting spring 230. Linking bar 212 is located distally to upper pivot axis 214 and lower pivot axis 216. Adjustment screw 228 extends internally from the top of linking bar 212 and contacts force limiting spring 230. Force limiting spring 230 is also in contact with linking bar upper pivot axis 224. In the configuration of FIGs. 8A-8C, the compressive force experienced by user's finger F is limited by use of adjustment screw 228, force limiting spring 230, and linking bar upper pivot axis 224. Linking bar upper pivot axis 224 can reversibly and linearly move against force limiting spring 230 when the force against linking bar upper pivot axis 324 exceeds a pre-load bias set by force limiting spring 230. Adjustment screw 228 also enables the aforementioned pre-load bias to be adjustably set by varying the amount of compression between adjustment screw 228 and linking bar upper pivot axis 224.

Although a force limiting means is optional in apparatus for extracting bodily fluid according to embodiments of the preset invention, such force limiting means can be useful for ensuring that an optimal compressive force is applied to various sized user's fingers. In addition, use of a force limiting means within its operative boundaries can serve to limit total compressive force to no more than, for example, 10N.

Upper arm assembly 210 and lower arm assembly 208 can be formed of suitable rigid material including, but not limited to, aluminum, steel, polystyrene, polycarbonate and polyester. Upper arm assembly 210 can be also constructed of flexible materials including, but not limited to, polypropylene such that upper arm assembly 210 bends when the compression force against user's finger F exceeds a predetermined limit.

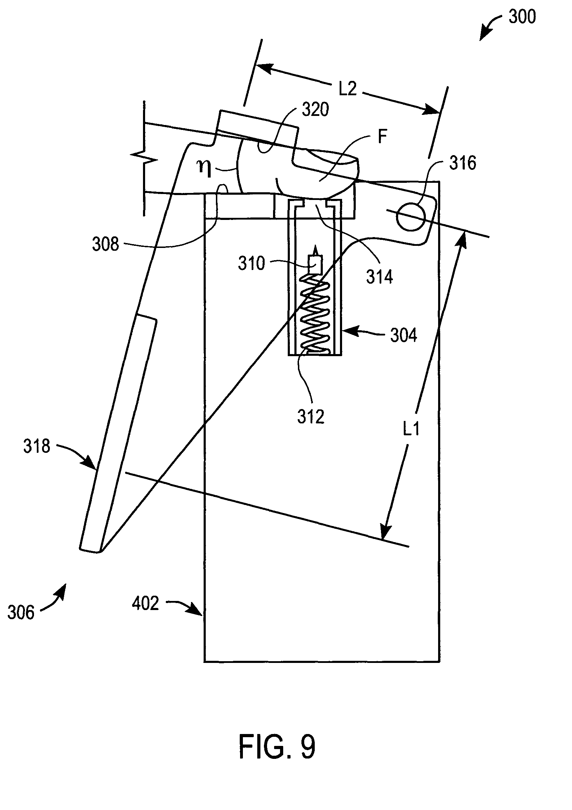

FIG. 9 depicts an apparatus 300 for extracting bodily fluid according to yet another embodiment of the present invention. Apparatus 300 (as well as apparatus 400 and apparatus 500 described below) applies compressive force to a user's finger F such that a tourniquet effect is applied to user's finger F causing sufficient blood to pool at the dermal tissue lancing site that bodily fluid extraction is successful.

Apparatus 300 includes a housing 302, a lancing mechanism 304 and a clamping mechanism 306. Housing 302 includes a means for measuring, analyzing and displaying an analyte concentration (not shown). Housing 302 also includes a lower compression surface 308. Lancing mechanism 304 is adjacent to lower compression surface 308, and includes a dermal tissue penetration member 310, a lancing spring 312 and an aperture 314 for the dermal tissue penetration member to pass through.

Clamping mechanism is pivotally attached to housing 302 by a pivot axis 316. Clamping mechanism 306 includes a lever arm 318 and an inner compression surface 320. Inner compression surface 320 can be made of a compliant material including, for example, rubber or foam and can be contoured to adapt to a shape of user's finger F. The angle η formed between inner compression surface 320 and lower compression surface 308 can range from about 0 degrees to about 90 degrees during operation of apparatus 300.

Inner compression surface 320 is in opposing relationship to lower compression surface 308 of housing 302. Lower compression surface 308 can be made, for example, of compliant material including rubber or foam and can be contoured to the shape of user's finger F.

Inner compression surface 320 and lower compression surface 308 are configured to apply a compressive force on user's finger F (in a manner similar to a tourniquet), when lever arm 318 and housing 302 are squeezed together (i.e., toward one another) and lever arm 318 rotates toward housing 302, thus decreasing angle η. In other words, the housing and clamping mechanism are operatively connected such that a user's finger inserted between the upper compression surface and inner compression surface is engaged with a compressive force when the lever arm and housing are squeezed together (i.e., towards one another). The squeezing can be accomplished, for example, manually by a user's hand. The squeezing action is an intuitive action to the user.

Clamping mechanism 306 applies a compressive force on a user's finger via mechanical advantage provided by the clamping mechanism's configuration. In the embodiment of FIG. 9, the mechanical advantage is the ratio of dimensions L1 and L2 (i.e., L1/L2). Therefore, the compressive force on a user's finger is beneficially greater than the force exerted on the lever arm and housing to squeeze them together. The mechanical advantage ratio of L1/L2 can be, for example, in the range of greater than 1 to 10.

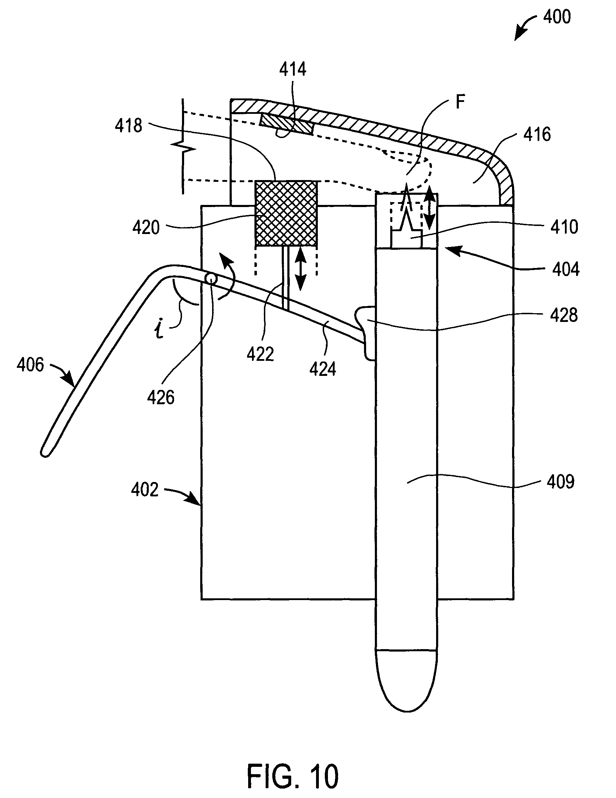

FIG. 10 depicts an apparatus 400 for extracting bodily fluid. Apparatus 400 includes a housing 402, a lancing mechanism 404 and clamping mechanism 406. Lancing mechanism 404 includes an aperture (not shown), a trigger 409 and a dermal tissue penetration member 410.

Clamping mechanism 406 includes an inner compression surface 414, a cavity 416, a lower compression surface 418 on a movable compression element 420, a vertical connector 422, and a lever arm 424. Lever arm 424 includes a pivot axis 426 and a trigger release 428.

Inner compression surface 414 is located within cavity 416. Lower compression surface 418 is also located within cavity 416 in an opposing relationship with inner compression surface 414. Compression element 420 is attached to lever arm 424 by vertical connector 422. The angle ι formed between lever arm 424 and housing 402 can range from about 0 degrees to about 90 degrees during operation of apparatus 400.

Inner compression surface 414 and lower compression surface 418 are configured to apply a compressive force on user's finger F (in a manner similar to a tourniquet), when lever arm 424 and housing 402 are squeezed together and lever arm 424 rotates toward housing 402, thus decreasing angle ι. The squeezing action is an intuitive action to the user and also serves to activate (i.e., fire) lancing mechanism 404 via trigger release 428.

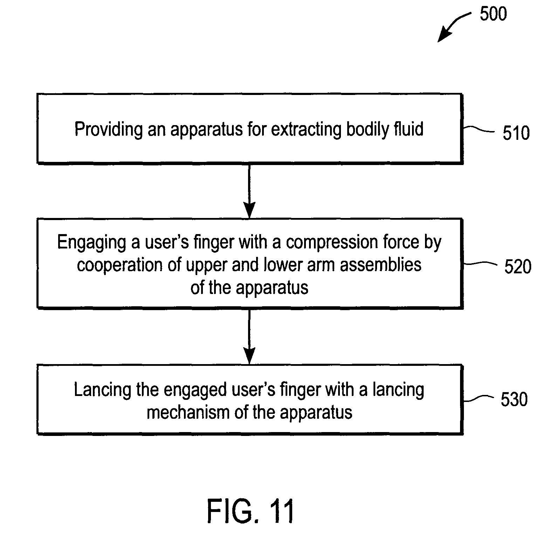

Referring to FIG. 11, a method 500 for extracting bodily fluid from a target site according to an exemplary embodiment of the present invention includes step 510 for providing an apparatus for extracting bodily fluid that includes (i) a housing; (ii) a lancing mechanism for lancing a target site attached to the housing and (iii) a clamping mechanism attached to the housing. In addition, the clamping mechanism of the apparatus includes upper and lower arm assemblies. Such apparatus have been described above (e.g., with respect to FIGs. 1 and 8A).

Next, a predetermined force is applied to the lower arm assembly with a user's finger such that the lower arm assembly is displaced from a first position to a second position, as set forth in step 520 of FIG. 11. Upon such displacement, the upper arm assembly and lower arm assembly cooperate to engage the user's finger with a compressive force that is greater than the predetermined user force (as described above with respect to, for example, FIG. 7C).

Subsequently, as set forth in step 530 of FIG. 11, a target site on the user's finger is lanced with the lancing mechanism, while the upper arm assembly and lower arm assembly are cooperating to engage the user's finger, whereafter the compressive force serves to extract a bodily fluid sample from the lanced target site.

It should be understood that various alternatives to the embodiments of the invention described herein may be employed in practicing the invention. It is intended that the following claims define the scope of the invention and that structures and methods within the scope of these claims and their equivalents be covered thereby

**Claims**

1. An apparatus for extracting bodily fluid, the apparatus comprising:

    a housing;

    a lancing mechanism for lancing a target site attached to the housing;

    a clamping mechanism attached to the housing, the clamping mechanism including:

        a lower arm assembly; and

        an upper arm assembly;

    wherein the lower arm assembly and upper arm assembly are operatively connected such that a when a user's finger applies a predetermined user force to the lower arm assembly, the lower arm assembly is displaced from a first position to a second position and the upper arm assembly and lower arm assembly cooperate to engage the user's finger with a compressive force that is greater than the predetermined user force; and

    wherein the lancing mechanism is configured to lance a target site on the user's finger while the upper arm assembly and lower arm assembly are cooperating to engage the user's finger, whereafter the compressive force serves to extract a bodily fluid sample from the lanced target site.

2. The apparatus of claim 1, wherein the lower arm assembly and upper arm assembly cooperate via mechanical advantage to engage the user's finger with the compressive force.

3. The apparatus of claim 1 or 2, wherein the clamping mechanism is pivotally attached to the housing and configured for one-handed operation of the apparatus.

4. The apparatus of claim 1, claim 2 or claim 3, wherein the lower arm assembly includes a pressure ring and the upper arm assembly includes a compression surface and wherein the pressure ring and compression surface cooperate to engage the user's finger with the compressive force.

5. The apparatus of any one of the preceding claims, further including means for limiting the compressive force.

6. The apparatus of claim 5, wherein the means for limiting the compressive force includes:

    a force limiting arm; and
    a force limiting spring,

    wherein the force limiting arm and force limiting spring are operatively connected to limit the compressive force.

7. The apparatus of claim 5, wherein the means for limiting the compressive force includes:

    an adjustment screw; and
    a force limiting spring,

    wherein the adjustment screw and force limiting spring are operatively connected to limit the compressive force.

8. The apparatus of any one of the preceding claims, wherein apparatus further includes a linking arm and the lower arm assembly and upper arm assembly are operatively connected by the linking arm.

9. The apparatus of any one of the preceding claims, wherein the predetermined force is less than approximately 6N and the compressive force is between 9N and 18N.

10. An apparatus for extracting bodily fluid, the apparatus comprising:

    a housing with a lower compression surface;
    a lancing mechanism for lancing a target site attached to the housing;
    a clamping mechanism attached to the housing, the clamping mechanism including:

        a lever; and
        an inner compression surface operatively aligned with the lower compression surface,

    wherein the housing and clamping mechanism are operatively connected such that a user's finger inserted between the upper compression surface and inner compression surface is engaged with a compressive force when the lever and housing are squeezed together.

11. The apparatus of claim 10 further including a trigger release, wherein the trigger release is configured to activate the lancing mechanism when the lever and housing are squeezed together.

12. The apparatus of claim 10 or 11, wherein lever arm and housing cooperate via mechanical advantage to produce the compressive force.

13. A method for extracting bodily fluid from a target site, the method comprising:

    providing an apparatus for extracting bodily fluid including:

        a housing;

        a lancing mechanism for lancing a target site attached to the housing;

        a clamping mechanism attached to the housing, the clamping mechanism including:

            a lower arm assembly; and

            an upper arm assembly;

    applying a predetermined force to the lower arm assembly with a user's finger such that the lower arm assembly is displaced from a first position to a second position and the upper arm assembly and lower arm assembly cooperate to engage the user's finger with a compressive force that is greater than the predetermined user force; and

lancing a target site on the user's finger while the upper arm assembly and lower arm assembly are cooperating to engage the user's finger, whereafter the compressive force serves to extract a bodily fluid sample from the lanced target site.

14. The method of claim 13, wherein the providing step further includes providing an apparatus for extracting bodily fluid that also includes means for limiting the compressive force and the applying step further includes the means for limiting the compressive force acting to limit the compressive force.

FIG. 1

100

FIG. 2A

FIG. 2B

FIG. 2C

FIG. 3A

FIG. 3B

FIG. 4

FIG. 5A

FIG. 5B

FIG. 6A

FIG. 6B

FIG. 7A

FIG. 7B

FIG. 7C

FIG. 8A

200

F

212

224

204

202

FIG. 8B

FIG. 8C

FIG. 9

FIG. 10

500

| Providing an apparatus for extracting bodily fluid | 510 |

↓

| Engaging a user's finger with a compression force by cooperation of upper and lower arm assemblies of the apparatus | 520 |

↓

| Lancing the engaged user's finger with a lancing mechanism of the apparatus | 530 |

FIG. 11